# EUROPEAN PATENT APPLICATION

(11) **EP 4 843 620 A1**
(43) Date of publication of application: **25.11.2026**
(21) Application number: 26180234.2
(22) Date of filing: 21.05.2026
(51) Int. Cl.: A61N 5/10

(54) **RADIATION TREATMENT PLAN OPTIMIZATION METHOD, APPARATUS, AND NON-TRANSITORY COMPUTER-READABLE MEDIUM**

(30) Priority: 23.05.2025 US 202519217016
(71) Applicant: Siemens Healthineers International AG, 6312 Steinhausen (CH)
(72) Inventor: KUUSELA, Esa, 00100 Helsinki (FI); BASIRI, Shahab, 00100 Helsinki (FI); MYLLYKOSKI, Mirko, 00100 Helsinki (FI); RUSANEN, Marko, 00100 Helsinki (FI); RUOKOKOSKI, Emmi, 00100 Helsinki (FI); PIETILA, Ville, 00100 Helsinki (FI); SABEL, Martin, 5405 Dättwil (CH)
(74) Representative: Mathisen & Macara LLP

(57) **Abstract**

A control circuit 101 optimizes a radiation treatment plan as a function of limiting velocity changes at at least one control point for a plurality of dynamic machine axes 202 (such as, but not limited to, movement of a radiation source 115, movement of a patient support apparatus 110, and/or movement of at least part of a radiation beam collimator 117) to provide an optimized radiation treatment plan 113. By one approach, the foregoing can include predicting a temporal duration of each selected control point interval and a velocity of each of the dynamic machine axes at each selected control point interval. By one approach, that prediction can comprise assuming that the temporal duration is determined by whichever one of the plurality of dynamic machine axes takes a longest time to move from a position of a previous control point to a position of a next control point when assumed to move with a maximum speed.

## Description

### TECHNICAL FIELD

These teachings relate generally to treating a patient's target volume with energy pursuant to an energy-based treatment plan and more particularly to optimizing an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

### SUMMARY

In one aspect, the present invention provides a method for optimizing a radiation treatment plan for a particular patient for use with a particular radiation treatment machine having a plurality of dynamic machine axes, the method comprising, by a control circuit, optimizing the radiation treatment plan as a function of limiting velocity changes at at least one control point for the plurality of dynamic machine axes to provide an optimized radiation treatment plan. The plurality of dynamic machine axes may be axes that are to be utilized during administration of the radiation treatment plan. The administration of the radiation treatment is not part of the claimed invention. The method for optimizing a radiation treatment plan may be as defined in claim 1. Optional features are specified in the dependent claims.

In another aspect, the present invention provides an apparatus for optimizing a radiation treatment plan, as defined in claim 11. Optional features are specified in the dependent claim.

In a further aspect, the present invention provides a non-transitory computer-readable medium for optimizing a radiation treatment plan, as defined in claim 13. Optional features are specified in the dependent claim.

### BRIEF DESCRIPTION OF DRAWINGS

Various needs in the foregoing regards are at least partially met through provision of the radiation treatment plan optimization method, apparatus, and non-transitory computer-readable medium described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a timing diagram as configured in accordance with various embodiments of these teachings;
FIG. 4 comprises a timing diagram as configured in accordance with various embodiments of these teachings; and
FIG. 5 comprises a timing diagram as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Generally speaking, these various embodiments can provide for optimizing a radiation treatment plan for a particular patient using a particular radiation treatment machine having a plurality of dynamic machine axes to be utilized during administration of the radiation treatment plan. By one approach, the foregoing can comprise a control circuit optimizing the radiation treatment plan as a function of limiting velocity changes at at least one control point for the plurality of dynamic machine axes to provide an optimized radiation treatment plan.

These teachings will accommodate any one or more of a variety of dynamic machine axes. Some examples include, but are not limited to, movement of a radiation source, movement of a patient support apparatus, and/or movement of at least part of a radiation beam collimator.

By one approach, the aforementioned optimization of the radiation treatment plan as a function of limiting velocity changes at at least one control point for the plurality of dynamic machine axes can include predicting a temporal duration of each selected control point interval and a velocity of each of the dynamic machine axes at each selected control point interval. By one approach, that prediction can comprise assuming that the temporal duration is determined by whichever one of the plurality of dynamic machine axes takes a longest time to move from a position of a previous control point to a position of a next control point when assumed to move with a maximum speed.

By one approach, these teachings will further accommodate calculating individual dynamic machine axis velocities by dividing a distance between positions in previous and next control points by a corresponding temporal duration.

By one approach, the aforementioned optimizing of the radiation treatment plan as a function of limiting velocity changes at at least one control point for the plurality of dynamic machine axes can further include calculating, based on axis acceleration and deceleration limits, a largest possible dynamic machine axis velocity change at each selected control point. In lieu of the foregoing or in combination therewith, optimizing the radiation treatment plan as a function of limiting velocity changes at at least one control point for the plurality of dynamic machine axes can further include penalizing dynamic machine axis velocity changes that exceed a maximum threshold at a single control point (or, in the alternative, configuring at least one optimization loop to prevent changes to control point interval durations that will change a velocity of at least one non-optimized dynamic machine axis beyond a threshold value).

By one approach, these teachings can comprise a non-transitory computer-readable medium having a computer program that itself comprises instructions that, when the computer program is executed by a computer, causes the computer to carry out optimizing a radiation treatment plan as a function of limiting velocity changes at at least one control point for a plurality of dynamic machine axes to provide an optimized radiation treatment plan.

So configured, these teachings can help to avoid unnecessary radiation treatment machine slowdowns by limiting velocity changes in at least some (or all) control points for at least some (or all) of the corresponding dynamic machine axes.

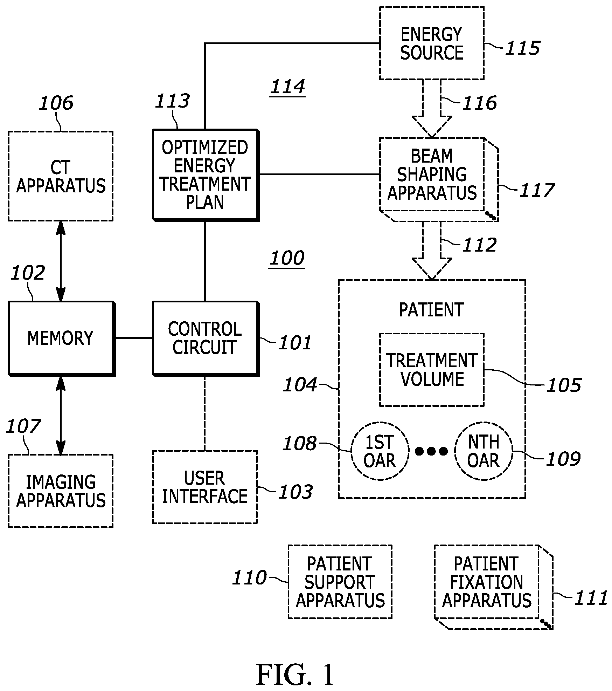

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM)))).

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

Accordingly, a radiation treatment platform (sometimes also referred to as a radiation treatment machine) has a plurality of dynamic machine axes including, but not limited to, movement of a radiation source 115, movement of a patient support apparatus 110, and movement of at least part of a radiation beam collimator that may comprise a part of the above-described beam shaping apparatus 117

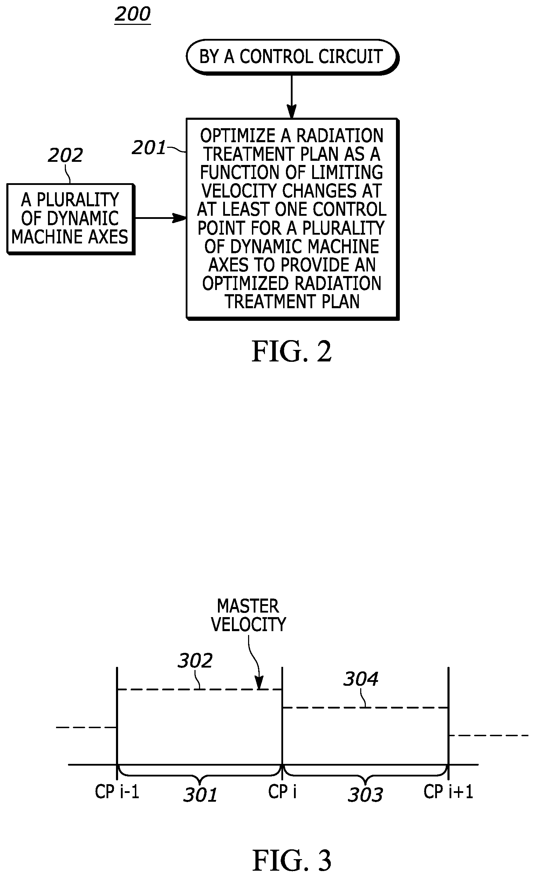

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan. More particularly, this process 200 can serve to optimize a radiation treatment plan for a particular patient using a particular radiation treatment machine having a plurality of dynamic machine axes to be utilized during administration of the radiation treatment plan.

When optimizing a radiation treatment plan, it can be helpful to create the plan in a manner that ensures that the radiation treatment plan is valid for physical implementation and can be in fact delivered with the selected particular radiation treatment machine. The applicant has determined that this can mean observing not only hard movement limits of various axes, but also dynamic limits, such as maximum speed limits, maximum acceleration/deceleration limits, and even limits for higher moments (including a maximum limit of the third derivative (jerk) and the fourth derivative (snap)). While a violation of these limits does not necessarily cause the plan to be physically untenable, ignoring such limits might cause a significant slowdown of the radiation treatment machine during treatment that, in turn, causes the overall treatment time to increase beyond a maximum treatment time identified by the user or otherwise.

By at least one prior art approach, each radiation treatment machine axis position is described in a sequence of control points that describe an ordered list of states that the radiation treatment machine must achieve. Between the control points, the position of the axis can be interpolated from a nearest control point. In order to follow this synchronous motion everywhere, however, the radiation treatment machine must either instantaneously change the axis velocities at every control point, or synchronously always first decelerate all axis to rest when reaching the control point and then accelerate back to the largest possible velocity. Neither of those options is feasible, however. As a result, the radiation treatment machine is typically allowed to move each axis asynchronously in the vicinity of the corresponding control point so long as that asynchronous motion does not cause a position error that is larger than a given tolerance (which could be, for example, 0.1 degrees for rotational axes and 0.1 mm for transversal axes). When that approach is not possible, however, the radiation treatment machine will automatically slow down and that can lead to an elongated treatment time.

With the foregoing in mind, at block 201, the control circuit 101 optimizes the radiation treatment plan as a function of limiting velocity changes at at least one control point for a plurality of dynamic machine axes 202 to provide an optimized radiation treatment plan. That plurality of dynamic machine axes 202 can include at least one, two, or all of movement of a radiation source 115, movement of a patient support apparatus 110, and/or movement of at least part of a radiation beam collimator 117 which may include rotation of the collimator itself and/or any other motion of the leaves or jaws of a multi-leaf collimator.

By one approach, optimizing the radiation treatment plan as a function of limiting velocity changes at at least one control point for the plurality of dynamic machine axes 202 can include predicting a temporal duration of each selected control point interval and a velocity of each of the dynamic machine axes at each selected control point interval. By one approach, predicting that temporal duration may comprise assuming that the temporal duration is determined by whichever one of the plurality of dynamic machine axes 202 takes a longest time to move from a position of a previous control point to a position of a next control point when assumed to move with a maximum speed permitted by the physical constraints of the radiation treatment machine.

This process 200 will further accommodate calculating individual dynamic machine access velocities by dividing a distance between positions in previous and next control points by a corresponding temporal duration. In lieu of the foregoing, or in combination therewith, this process 200 will accommodate optimizing the radiation treatment plan as a function of limiting velocity changes at at least one control point for the plurality of dynamic machine axes 202 by calculating, based on axis acceleration and deceleration limits, a largest possible dynamic machine axis velocity change at each selected control point.

By one approach, optimizing the radiation treatment plan as a function of limiting velocity changes at at least one control point for the plurality of dynamic machine axes 202 can further include penalizing dynamic machine axes velocity changes in the optimization algorithm that exceed a maximum threshold at a single control point. In lieu of the foregoing, or in combination there with, these teachings will also accommodate configuring at least one optimization loop to prevent changes to control point interval durations that will change a velocity of at least one non-optimized dynamic machine axis beyond a threshold value.

So configured, these teachings can serve to avoid unnecessary radiation treatment machine slowdowns by, at least in part, limiting velocity changes in each control point for some or, if desired, all of the dynamic machine axes. For axes whose positions are changed during the optimization, this can be done by (1) predicting the duration of each control point interval and a velocity of each axis at every control point interval, (2) based on axis acceleration and deceleration limits, calculating the largest possible velocity change at each control point, and (3) introducing a step or optimization objective to the optimization loop that prevents or penalizes changes in axis velocities that are too large in a single control point. For axes whose positions are not optimized, the later step can be replaced by introducing a step or optimization objective to the optimization loop that prevents the optimized axes to change in a way that would introduce changes to the control point interval durations where the resultant velocity of the non-optimized axis is changed too much (for example, in excess of a predetermined threshold value).

Predicting the duration of a given control point interval can be done in any of a variety of ways. By one approach, these teachings will assume that the duration is determined by whichever axis takes the longest time to move from the position of the previous control point to the position of the next control point when assumed to move with its maximum available speed. A more accurate prediction may obtained if also the effect of the finite acceleration is taken into account, where that acceleration cannot be avoided (for example, at the beginning and at the end of a field where all machine axes are starting (or ending) at rest). Another approach for the prediction of the control point interval durations can be based on collecting data from various plans and training a prediction model to output the desired information.

Once the durations of the control point intervals are determined, by one approach, the individual axis velocities can be calculated by dividing the distance between positions in previous and next control points by the corresponding duration.

By one approach, a largest possible velocity change can be deduced as follows. If Δ*vⱼᵢ* is the velocity change of axis j at control point i, it has the following constraints: $\left|Δ{v}_{ji}\right| < \sqrt{8 {T}_{j} {a}_{j}^{max}}$ where *Tⱼ* is the tolerance associated with axis j and ${a}_{j}^{max}$ is the maximum acceleration/deceleration of axis j.

When there are other constraints that can or should be taken into account (for example, a constraint that is based on treatment machine controller logic), such constraints can also be taken also into account if desired when determining the largest possible velocity change.

If desired, the limit can be enforced in the optimization by introducing an additional term *C_{sd}*(*X*) to the cost function *Cₜₒₜ*(*X*): ${C}_{tot} \left(X\right) = {C}_{dos} \left(F\left(x\right)\right) + {C}_{lp} \left(X\right) + {C}_{sd} \left(X\right) ,$ where vector *X* represents the axis positions to be optimized, and *C_{dos}*(*F*(*X*)) and *Cₗₚ*(*X*) are the standard dosimetrical (depending on the axis positions via dose calculation F(X)) and leaf terms used in the optimization (where the leaf term can contain components from trying to reduce treatment time, control aperture shape, and so forth)). One choice for *C_{sd}* is to consider it to be a sum of individual control point contributions (and to penalize velocity change violations separately for every axis): ${C}_{sd} \left(X\right) = {\sum }_{ij} {c}_{j} \left(Δ{v}_{ij}\right) .$

The cost component of individual axis can be chosen in any of a plurality of ways. By one approach, it may be beneficial to require the following properties: ${c}_{j} \left(Δv\right) = \left\{\begin{matrix}0 & if \left|Δv\right| + ϵ < \sqrt{4 {T}_{j} {a}_{j}^{max}} \\ > 0 & otherwise\end{matrix},\right.$ where *ε* is a small parameter that provides for leaving some tolerance. This cost term can introduce a bias for the axis locations in a given control point i to move towards a direction that will reduce the velocity difference at that control point (that is, the difference between the velocity before and after that control point).

Further details that comport with these teachings will now be presented. It will be understood that the specific details of these examples are intended to serve an illustrative purpose and are not intended to suggest any particular limitations with respect to these teachings.

Avoiding unduly slowing down may be slightly more complicated for an axis that is not optimized. That is at least due to the position of that axis not being directly changed. Nevertheless, a requirement to avoid slowdowns will create a restriction regarding how the durations of the control points can change. For example, in a typical optimization scenario, the gantry positions are defined up-front. If the velocity of the gantry changes too much at a given control point, the velocity difference can be reduced by adjusting the duration of the previous and the next control point interval. This, in turn, requires that one or more optimized axis positions be changed so that they either become the slowest axis and thus require a longer time to move the planned distance, or, if they were already the slowest axis, the distance to travel gets shorter. For stability, it may be useful to restrict such manipulations to the latter instance.

So configured, these teachings allow a reduction of the treatment time for a given radiation treatment plan by reducing the need to slow down the plan delivery that are due to restrictions from finite acceleration speed.

Ideally, a given machine axis would move with constant speed within a control point interval and then change velocity instantaneously at the control point location. Such performance, however, is not a reasonable expectation.

To allow synchronous motion, the present teachings can provide for deducing a master velocity, which velocity is such that none of the axes need to move faster than its particular maximum velocity. In those regards, and for these purposes, a master velocity *v* is a scalar (or speed) that is either zero or a positive number. In addition, the master velocity is a maximum of normalized maximum velocities for each individual axis. The axis velocities v can be normalized by dividing them by the travel distance *l* within the control point interval (with special handling, if desired, for the case when a particular axis does not move): $v = \frac{v}{l} .$

By one approach, a master velocity can be defined separately for each control point interval. FIG. 3 presents an illustrative example in these regards, where a first control point interval 301 has a corresponding first master velocity 302 while a subsequent second control point interval 303 has a corresponding second master velocity 304 that is smaller than the first master velocity 302.

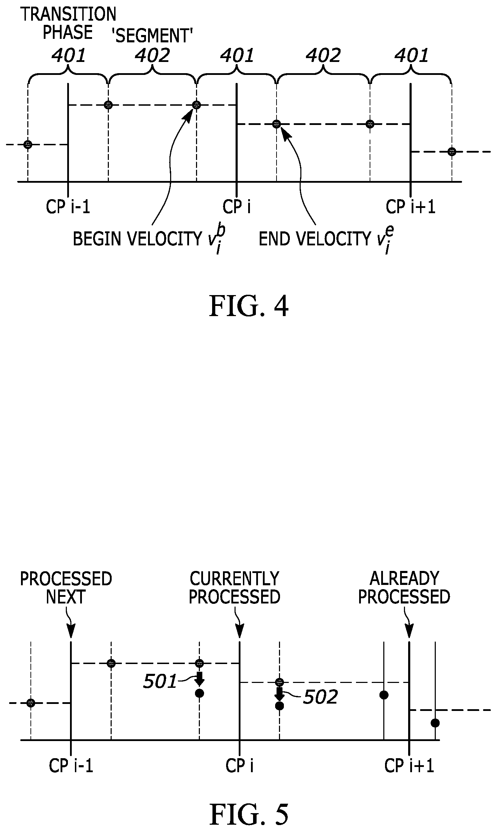

With reference to FIG. 4, each control point interval can be divided into a transition phase 401 and a segment 402. The transition phase 401 extends on either side of a control point. By one approach, the transition phase 401 may extend timewise by a same amount in both the previous and the next interval, where the axis can move asynchronously.

One segment 402 is the central region within a given control point interval where the axes are moving synchronously.. These transition phases 401 can be first initialized to be within that maximum width, and they can each have a beginning (or entry) velocity and an ending (or exit) velocity that cannot be larger than master velocities of either the previous control point interval or the next following control point interval.

By one approach, following the aforementioned initialization, the transition phases can be refined (starting from the end in this example) using two alternating steps. In a first step, maximum-deceleration reductions are done to the master velocity (including the beginning and ending velocity of the previous transition phase ("previous" meaning being previous in the actual treatment order)). A second step can provide for reducing the beginning and ending velocity of that next transition phase so that the transition can happen without unduly large errors and without exceeding the maximum size of the transition phase.

With reference to FIG. 5, once the necessary deceleration of a next interval is checked/fixed, these teachings will optionally accommodate next proceeding with respect to the actual transition phase. In particular, and as shown generally in FIG. 5, the beginning and ending velocities might need a downward adjustment 501 and 502 in order that the transition phase satisfy the following condition:
During asynchronous motion the position of the axis *j* does not differ from the planned position by more than a set tolerance *T^{j}*.

Transition zones can be handled as planned while reducing the beginning and ending velocities of the next transition phase as described above, except if the velocity at the beginning of the phase is smaller than the beginning velocity, in which case the transition phase can be recalculated by reducing the ending velocity accordingly (such that the width is not updated).

Further aspects of these teachings are provided by the subject matter of the following clauses (where it will be understood that any of these clauses can be combined with any one of more of the other clauses as desired).

Clause 1. A method for optimizing a radiation treatment plan for a particular patient using a particular radiation treatment machine having a plurality of dynamic machine axes to be utilized during administration of the radiation treatment plan, the method comprising: by a control circuit: optimizing the radiation treatment plan as a function of limiting velocity changes at at least one control point for the plurality of dynamic machine axes to provide an optimized radiation treatment plan.

Clause 2. The method of clause 1 wherein the plurality of dynamic machine axes include at least one of: movement of a radiation source; movement of a patient support apparatus; movement of at least part of a radiation beam collimator.

Clause 3. The method of clause 1 wherein the plurality of dynamic machine axes include at least two of: movement of a radiation source; movement of a patient support apparatus; movement of at least part of a radiation beam collimator.

Clause 4. The method of clause 1 wherein optimizing the radiation treatment plan as a function of limiting velocity changes at at least one control point for the plurality of dynamic machine axes includes predicting a temporal duration of each selected control point interval and a velocity of each of the dynamic machine axes at each selected control point interval.

Clause 5. The method of clause 4 wherein predicting the temporal duration comprises assuming that the temporal duration is determined by whichever one of the plurality of dynamic machine axes takes a longest time to move from a position of a previous control point to a position of a next control point when assumed to move with a maximum speed.

Clause 6. The method of clause 4 further comprising: calculating individual dynamic machine axis velocities by dividing a distance between positions in previous and next control points by a corresponding temporal duration.

Clause 7. The method of clause 4 wherein optimizing the radiation treatment plan as a function of limiting velocity changes at at least one control point for the plurality of dynamic machine axes further includes calculating, based on axis acceleration and deceleration limits, a largest possible dynamic machine axis velocity change at each selected control point.

Clause 8. The method of clause 7 wherein optimizing the radiation treatment plan as a function of limiting velocity changes at at least one control point for the plurality of dynamic machine axes further includes penalizing dynamic machine axis velocity changes that exceed a maximum threshold at a single control point.

Clause 9. The method of clause 7 wherein optimizing the radiation treatment plan as a function of limiting velocity changes at at least one control point for the plurality of dynamic machine axes further includes configuring at least one optimization loop to prevent changes to control point interval durations that will change a velocity of at least one non-optimized dynamic machine axis beyond a threshold value.

Clause 10. An apparatus for optimizing a radiation treatment plan for a particular patient using a particular radiation treatment machine having a plurality of dynamic machine axes to be utilized during administration of the radiation treatment plan, the apparatus comprising: a control circuit configured to optimize the radiation treatment plan as a function of limiting velocity changes at at least one control point for the plurality of dynamic machine axes to provide an optimized radiation treatment plan.

Clause 11. The apparatus of clause 10 wherein the plurality of dynamic machine axes include at least one of: movement of a radiation source; movement of a patient support apparatus; movement of at least part of a radiation beam collimator.

Clause 12. The apparatus of clause 10 wherein the plurality of dynamic machine axes include at least two of: movement of a radiation source; movement of a patient support apparatus; movement of at least part of a radiation beam collimator.

Clause 13. The apparatus of clause 10 wherein the control circuit is configured to optimize the radiation treatment plan as a function of limiting velocity changes at at least one control point for the plurality of dynamic machine axes by predicting a temporal duration of each selected control point interval and a velocity of each of the dynamic machine axes at each selected control point interval.

Clause 14. The apparatus of clause 13 wherein the control circuit is configured to predict the temporal duration by assuming that the temporal duration is determined by whichever one of the plurality of dynamic machine axes takes a longest time to move from a position of a previous control point to a position of a next control point when assumed to move with a maximum speed.

Clause 15. The apparatus of clause 13 wherein the control circuit is further configured to: calculate individual dynamic machine axis velocities by dividing a distance between positions in previous and next control points by a corresponding temporal duration.

Clause 16. The apparatus of clause 13 wherein the control circuit is configured to optimize the radiation treatment plan as a function of limiting velocity changes at at least one control point for the plurality of dynamic machine axes by calculating, based on axis acceleration and deceleration limits, a largest possible dynamic machine axis velocity change at each selected control point.

Clause 17. The apparatus of clause 16 wherein the control circuit is configured to optimize the radiation treatment plan as a function of limiting velocity changes at at least one control point for the plurality of dynamic machine axes by penalizing dynamic machine axis velocity changes that exceed a maximum threshold at a single control point.

Clause 18. The apparatus of clause 16 wherein the control circuit is configured to optimize the radiation treatment plan as a function of limiting velocity changes at at least one control point for the plurality of dynamic machine axes by configuring at least one optimization loop to prevent changes to control point interval durations that will change a velocity of at least one non-optimized dynamic machine axis beyond a threshold value.

Clause 19. A non-transitory computer-readable medium for optimizing a radiation treatment plan for a particular patient using a particular radiation treatment machine having a plurality of dynamic machine axes to be utilized during administration of the radiation treatment plan comprising instructions stored thereon, that when executed on a processor, perform the steps of optimizing the radiation treatment plan as a function of limiting velocity changes at at least one control point for the plurality of dynamic machine axes to provide an optimized radiation treatment plan.

Clause 20. The non-transitory computer-readable medium of clause 19 wherein the plurality of dynamic machine axes include each of: movement of a radiation source; movement of a patient support apparatus; movement of at least part of a radiation beam collimator.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

## Claims

1. A method for optimizing a radiation treatment plan for a particular patient using a particular radiation treatment machine having a plurality of dynamic machine axes to be utilized during administration of the radiation treatment plan, the method comprising:
by a control circuit:
optimizing the radiation treatment plan as a function of limiting velocity changes at at least one control point for the plurality of dynamic machine axes to provide an optimized radiation treatment plan.

2. The method of claim 1 wherein the plurality of dynamic machine axes include at least one of:
movement of a radiation source;
movement of a patient support apparatus;
movement of at least part of a radiation beam collimator.

3. The method of claim 1 wherein the plurality of dynamic machine axes include at least two of:
movement of a radiation source;
movement of a patient support apparatus;
movement of at least part of a radiation beam collimator.

4. The method of claim 1 wherein the plurality of dynamic machine axes include each of:
movement of a radiation source;
movement of a patient support apparatus;
movement of at least part of a radiation beam collimator.

5. The method of any one of claims 1 to 4 wherein optimizing the radiation treatment plan as a function of limiting velocity changes at at least one control point for the plurality of dynamic machine axes includes predicting a temporal duration of each selected control point interval and a velocity of each of the dynamic machine axes at each selected control point interval.

6. The method of claim 5 wherein predicting the temporal duration comprises assuming that the temporal duration is determined by whichever one of the plurality of dynamic machine axes takes a longest time to move from a position of a previous control point to a position of a next control point when assumed to move with a maximum speed.

7. The method of claim 5 or 6 further comprising:
calculating individual dynamic machine axis velocities by dividing a distance between positions in previous and next control points by a corresponding temporal duration.

8. The method of any one of claims 1 to 7 wherein optimizing the radiation treatment plan as a function of limiting velocity changes at at least one control point for the plurality of dynamic machine axes further includes calculating, based on axis acceleration and deceleration limits, a largest possible dynamic machine axis velocity change at each selected control point.

9. The method of claim 8 wherein optimizing the radiation treatment plan as a function of limiting velocity changes at at least one control point for the plurality of dynamic machine axes further includes penalizing dynamic machine axis velocity changes that exceed a maximum threshold at a single control point.

10. The method of any one of claims 1 to 9 wherein optimizing the radiation treatment plan as a function of limiting velocity changes at at least one control point for the plurality of dynamic machine axes further includes configuring at least one optimization loop to prevent changes to control point interval durations that will change a velocity of at least one non-optimized dynamic machine axis beyond a threshold value.

11. An apparatus for optimizing a radiation treatment plan for a particular patient using a particular radiation treatment machine having a plurality of dynamic machine axes to be utilized during administration of the radiation treatment plan, the apparatus comprising:
a control circuit configured to optimize the radiation treatment plan as a function of limiting velocity changes at at least one control point for the plurality of dynamic machine axes to provide an optimized radiation treatment plan.

12. The apparatus of claim 11 wherein the apparatus is configured to perform the method of any one of claims 2 to 4 and/or wherein the control circuit is configured to perform the method of any one of claims 5 to 10.

13. A non-transitory computer-readable medium for optimizing a radiation treatment plan for a particular patient using a particular radiation treatment machine having a plurality of dynamic machine axes to be utilized during administration of the radiation treatment plan comprising instructions stored thereon, that when executed on a processor, perform the steps of optimizing the radiation treatment plan as a function of limiting velocity changes at at least one control point for the plurality of dynamic machine axes to provide an optimized radiation treatment plan.

14. The non-transitory computer-readable medium of claim 13 wherein the instructions, when executed on a processor, perform the method of any one of claims 2 to 10.
